# EUROPÄISCHE PATENTSCHRIFT

(11) **EP 0 885 622 B1**
(45) Veröffentlichungstag und Bekanntmachung des Hinweises auf die Patenterteilung: **18.10.2000**
(21) Anmeldenummer: 98106065.0
(22) Anmeldetag: 02.04.1998
(51) Int. Cl.: A61M 13/00

(54) **Insufflationsnadel**
Inssuflation needle
Aiguille à insufflation

(30) Priorität: 23.06.1997 DE 19726579
(43) Veröffentlichungstag der Anmeldung: 23.12.1998
(73) Patentinhaber: WISAP GESELLSCHAFT FÜR WISSENSCHAFTLICHEN APPARATEBAU MBH, 82054 Sauerlach b. München (DE)
(72) Erfinder: Semm, Horst, 82031 Grünwald (DE)
(74) Vertreter: Klunker . Schmitt-Nilson . Hirsch

(56) Entgegenhaltungen:
- EP-A- 0 339 945
- WO-A-96/01132
- US-A- 5 290 276

## Beschreibung

Die Erfindung betrifft Insufflationsnadeln, aufweisend:
einen Basiskörper mit einer Einströmöffnung;
eine an dem Basiskörper angebrachte, hohle Insufflationslanze mit einer Ausströmöffnung in ihrem von dem Basiskörper entfernten Endbereich, die durch die Insufflationslanze und einen Kanal in dem Basiskörper mit der Einströmöffnung in Verbindung steht;
eine relativ zu dem Basiskörper und Insufflationlanze längsverschiebbar und lösbar angebrachte Kanüle, in der die Insufflationslanze aufgenommen ist, wobei die Insufflationslanze federnd so vorgespannt ist, daß im unbelasteten Zustand ihre Ausströmöffnung unabgedeckt von der Kanüle ist und daß im belasteten Zustand die Insufflationslanze in die Kanüle zurückdrückbar ist.

Derartige Insufflationsnadeln wurden bereit vor dem Anmeldetag des vorliegenden Patents beispielsweise von der Inhaberin vertrieben. Solche Insufflationsnadeln werden auch Veress-Nadel genannt. Es handelt sich dabei um eine Spezialkanüle zur Insufflation von Gas oder Flüssigkeit insbesondere in die menschliche Bauchhöhle.

Insbesondere bei der minimal invasiven Chirurgie, beispielweise der Laparoskopie und der Pelviskopie, bei der ohne eine großflächige Öffnung des Bauchraums Operationen im Bauchraum und am Unterleib durchgeführt werden können, finden Insufflationsnadeln ihre regelmäßige Anwendung. Bei einer Operation wird häufig zuerst mit der Insufflationsnadel in die Punktionsstelle an der Bauchdecke eingestochen. Dazu ist die Kanüle an ihrem vorderen Ende abgeschrägt und zugespitzt. Die Insufflationsnadel wird beim Einstechen an der Kanüle gehalten. Die in der Kanüle aufgenommene Insufflationslanze berührt zuerst die Bauchdecke und wird durch eine Weiterbewegung der Kanüle gegen die Vorspannung in die Kanüle zurückgedrückt, bis sie sich im wesentlichen völlig in der Kanüle befindet. Erst dann sticht beim Weiterbewegen die Kanüle in die Bauchdecke ein und durch diese hindurch.

Nach dem Durchstoßen der Bauchdecke wirkt keine Kraft mehr gegen die Insufflationslanze, so daß diese in Folge der Vorspannung wieder nach vorne aus der Kanüle geschoben wird. Der Operateur kann an der gleichzeitigen Bewegung des Basiskörpers erkennen, daß das Durchstechen der Bauchdecke abgeschlossen ist. Im Anschluß daran beginnt das Insufflieren des Bauchraums mit einem geeigneten Gas wie Kohlendioxid oder Lachgas (CO₂, NO₂). Mit einem Trokar, durch den der eigentliche Eingriff erfolgt, wird dann durch die Bauchdecke eingestochen.

Die Insufflationsnadel ist regelmäßig bereits während des Einstechens mit der Insufflationsgaszufuhr verbunden, und der Gegendruck, gegen den die Insufflationsgasversorgung arbeiten muß, wird von dieser angezeigt und kann eine sehr sensible Angabe darüber liefern, ob die Bauchdecke bereits durchstochen ist oder nicht. Die Ausströmöffnung der Insufflationslanze ist während des Einstechens von der Kanüle abgedeckt, was ein Strömen des Gases weitestgehend verhindert. Außerdem wirkt das umliegende Gewebe als zusätzliche Abdichtung. Der Strömungswiderstand nimmt mit dem Austreten der Lanze aus der Kanüle bedeutlich ab, bis er schließlich bei voll freigegebener Ausströmöffnung einen Minimalwert erreicht. Ein nicht zu überschreitender Innendruck im Bauchraum von etwa 12 bis 14 mmHg einerseits und Strömungsverluste in der Insufflationslanze von bis zu 8 mmHg bei einem Durchfluß von 1 Liter/min andererseits begrenzen die Möglichkeiten für diese Art der Überwachung des Durchstoßens der Bauchdecke. Da der intraabdominale Druck, d.h. der Druck im Bauchraum bzw. der Bauchhöhle, nicht beliebig erhöht werden kann, ist ein niedriger Strömungswiderstand der Insufflationsnadel für eine präzise Gegendrucküberwachung des Durchstechens erforderlich.

Insufflationsnadeln werden nach dem Gebrauch gereinigt und für eine neue Verwendung sterilisiert. Insufflationslanzen können aber bei der Handhabung und insbesondere beim Reinigen leicht beschädigt werden.

Dabei kommt es typisch zu Verbiegungen der Insufflationslanze oder der Kanüle insbesondere an derem vorderen Ende. Während Kanülen, die üblicherweise mittels einer Schraubverbindung an einer Befestigungshülse an dem Basiskörper befestigt sind, ersetzt werden können, führt eine Beschädigung an der Insufflationslanze, die in dem Basiskörper eingelötet ist, zu Unbrauchbarkeit der Insufflationsnadel als Ganzes.

Darüberhinaus ist das Reinigen und insbesondere das Sterilisieren von Insufflationsnadeln relativ problematisch. Blut und Geweberückstände, die in der Passage durch den Basiskörper und die Insufflationslanze und in der Kanüle zurückgeblieben sind, können nur durch intensives Durchspülen entfernt werden. Blut und Geweberückstände in der langen Passage durch den Basiskörper und die Insufflationslanze sind selbst durch Spülen nur äußerst schwer zu entfernen. Dazu kommt, daß die Schraubenfeder, welche die federnde Vorspannung liefert, am Basiskörper festgelegt ist, so daß auch eine Reinigung und Sterilisierung der Feder und des entsprechenden Basiskörperbereichs nur schwer möglich ist.

Der Erfindung liegt deshalb die Aufgabe zugrunde, eine Insufflationsnadel bereitzustellen, die mit geringem Aufwand zuverlässig gereinigt und sterilisiert werden kann und die weniger beschädigungsanfällig ist.

Zur Lösung dieser Aufgabe weist die vorangehend genannte Insufflationsnadel eine Insufflationslanze auf, die mit einer lösbaren Befestigungseinrichtung an dem Basiskörper befestigt ist. Die Reinigung des Basiskörpers ist so wesentlich erleichtert und auch die Reinigung der Insufflationslanze ist einfacher, da nicht die zusammenhängende Passage durch den Basiskörper und die Insufflationslanze als Ganzes gereinigt werden muß, sondern die Passagen einzeln gereinigt werden können. So sind Ecken und Kanten, an denen Verschmutzungen besonders schwierig zu beseitigen sind, leichter zugänglich oder vermieden. Zudem kann eine beschädigte Insufflationslanze leichter ausgetauscht werden und führt nicht zur Unbrauchbarkeit der gesamten Insufflationsnadel.

Vorzugsweise ist die Insufflationslanze mit einem Anschlag für ein Vorspannelement versehen, das die federnde Vorspannung liefert. Nach dem Lösen der Insufflationslanze von dem Basiskörper ist auch das Vorspannelement von dem Basiskörper frei und kann zur Reinigung entfernt werden. Außerdem kann die Befestigungshülse, an der der zweite Anschlag für das Vorspannelement vorgesehen ist, dann ebenfalls entfernt werden und separart gereinigt werden. Bei dem Vorspannelement handelt es sich vorzugsweise um eine Schraubenfeder.

Vorzugsweise ist an der Insufflationslanze ein Teil der Befestigungseinrichtung durch Laserschweißen befestigt. An der Insufflationslanze kann eine Buchse befestigt sein, an der der eine Teil der Befestigungseinrichtung vorgesehen ist. Diese Art der Befestigung der Insufflationslanze an der Befestigungseinrichtung ist eine besonders einfache, schnelle und kostengünstige Art der Verbindung. Man kann dabei sehr saubere Übergänge reproduzierbar und eventuell automatisiert herstellen, was die Strömungsverhältnisse an dem Übergang zur Insufflationslanze wesentlich verbessert. Es können auch andere Verbindungen wie Löten oder Kleben vorgesehen sein. Die Befestigungseinrichtung kann an die Insufflationslanze, beispielsweise aus einem Kunststoffmaterial angegossen sein. Die Herstellung der Insufflationsnadel ist besonders einfach, wenn der Anschlag durch die Buchse gebildet ist.

Es ist bevorzugt, den Kanal in dem Basiskörper durchgehend bis zur Befestigungseinrichtung oder der Insufflationslanze mit einem Strömungsquerschnitt auszubilden, der größer ist als der Strömungsquerschnitt der Insufflationslanze. Dadurch kann der Strömungswiderstand der Insufflationslanze deutlich gesenkt werden. Aus fertigungstechnischen Gründen ist bei den bekannten Insufflationsnadeln, bei denen die Insufflationslanze in dem Basiskörper eingelötet ist, der Kanal bereits deutlich vor dem Ende des Basiskörpers praktisch auf den Durchmesser der Insufflationslanze verjüngt, um einerseits bei der Herstellung ein Durchbohren bis zum Ende zu vermeiden, und andererseits, um das Verlöten der Insufflationslanze zu vereinfachen. Durch den größeren Strömungsquerschnitt über eine längere Strecke verglichen mit konventionellen Insufflationnadeln läßt sich der Strömungswiderstand der Isufflationsnadel je nach Länge der Insufflationslanze um bis zu 10% oder mehr veringern. Es wird daraufhingewiesen, daß dieses Merkmal für sich alleine betrachtet, ohne die zusätzlichen Merkmale des kennzeichnenden Teils von Anspruch 1 und der weiteren Ausgestaltungen davon oder mit nur einem Teil dieser Merkmale für sich alleine als erfinderisch angesehen wird. Der Kanal durch den Basiskörper kann eine durch diesen hindurchgehende Bohrung konstanten Querschnitts sein. Das erleichtert die Reinigung des Basiskörpers und führt zu einer Kosteneinsparung bei der Herstellung.

Bei der Befestigungseinrichtung kann es sich um eine Schraubverbindung oder um eine Schnellverbindungseinrichtung handeln. Das männliche Teil der Befestigungseinrichtung kann dabei sowohl an der Insufflationslanze als auch am Basiskörper vorgesehen sein. Es können vielerlei Schnellverbindungen verwendet werden, beispielsweise Renkverbindungen wie ein Bajonettverschluß oder eine andere Schnellverbindung beispielsweise eine "Luer Lock" oder LL-Verbindung, bei der es sich um ein zweigängiges Steilgewinde handelt. Zum schnelleren miteinander Verbinden des Basiskörpers und der Insufflationslanze kann der Befestigungseinrichtung am Basiskörper oder an der Insufflationslanze ein Einführkonus zugeordnet sein.

Vorzugsweise ist die Kanüle mit einer lösbaren Befestigungseinrichtung an einem Kanülenträger befestigt, mit dem die Kanüle an dem Basiskörper angebracht ist.

Die Erfindung betrifft ferner eine Insufflationslanze gemäß Anspruch 13 der sowie ein Austauschset mit Kanüle und mit Insufflationslanze gemäß Anspruch 14 und vorzugsweise mit Vorspannelement für die federnde Vorspannung der erfindungsgemäßen Insufflationsnadel. Die Insufflationslanze bzw. die Einzelteile des Austauschssets können steril verpackt sein. Sie können insbesondere als Einwegprodukte vorgesehen sein. Letzteres hat den Vorteil, daß das arbeitsaufwendige Reinigen dieser Teile völlig entfällt und lediglich das Reinigen des Basiskörpers erforderlich ist. Das Klinikpersonal wird dadurch entlastet, und das große Risiko für das Klinikpersonal, sich durch Stiche mit infizierten Nadeln zu Infizieren, ist beseitigt.

Die Erfindung wird nachfolgend anhand zeichnerisch dargestellter Ausführungsbeispiele noch näher erläutert. Es zeigen:
- Fig. 1: die Ansicht einer Insufflationsnadel;
- Fig. 2: eine Insufflationsnadel im auseinandergezogenen Zustand und zum Teil geschnitten ; und
- Fig. 3: eine alternative Ausführungsform einer lösbaren Befestigungseinrichtung zum Teil im Schnitt.

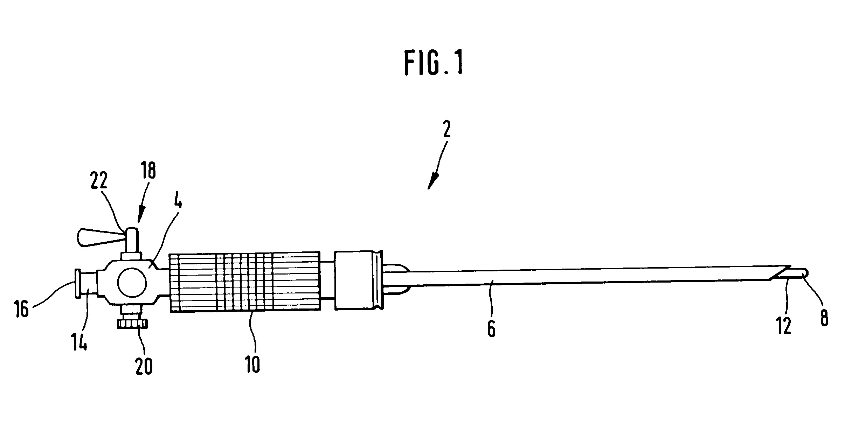

In Fig. 1 ist eine Insufflationsnadel 2 mit einem Basiskörper 4, einer Kanüle 6 und einer innerhalb der Kanüle 6 angeordneten Insufflationslanze 8 gezeigt. Die Kanüle 6 ist an einem Kanülenträger 10 befestigt, mit dem die Kanüle 6 an dem Basiskörper 4 angebracht ist. Der Kanülenträger 10 besitzt eine zylinderische Form und hat eine längs und quer geriffelte Außenoberfläche. Er dient gleichzeitig zum Greifen der Kanüle 6 und der Insufflationsnadel 2 beim Einstechen.

Basiskörper 4 und Insufflationslanze 8 sind relativ zu der Kanüle 6 und dem Trägerkörper 10 längsverschiebbar, wobei sie federnd so vorgespannt sind, daß im unbelasteten Zustand eine Ausströmöffnung 12 am vorderen Endebereich der Insufflationslanze 8 von der Kanüle 6 unabgedeckt ist. Wenn von vorne gegen die Insufflationslanze 8 gedrückt wird, kann diese in die Kanüle 6 zurückgedrückt werden, so daß die Ausströmöffnung 12 abgedeckt ist.

An dem Basiskörper 4 befindet sich an dem der Kanüle 6 und der Insufflationslanze 8 entgegengesetzten Ende ein Anschlußansatz 14 mit einer Einströmöffnung 16, und ein Absperrventil 18. Durch Lösen einer Mutter 20 kann ein Ventilkücken 22 beispielsweise zu Reinigungszwecken aus dem Basiskörper 4 entfernt werden.

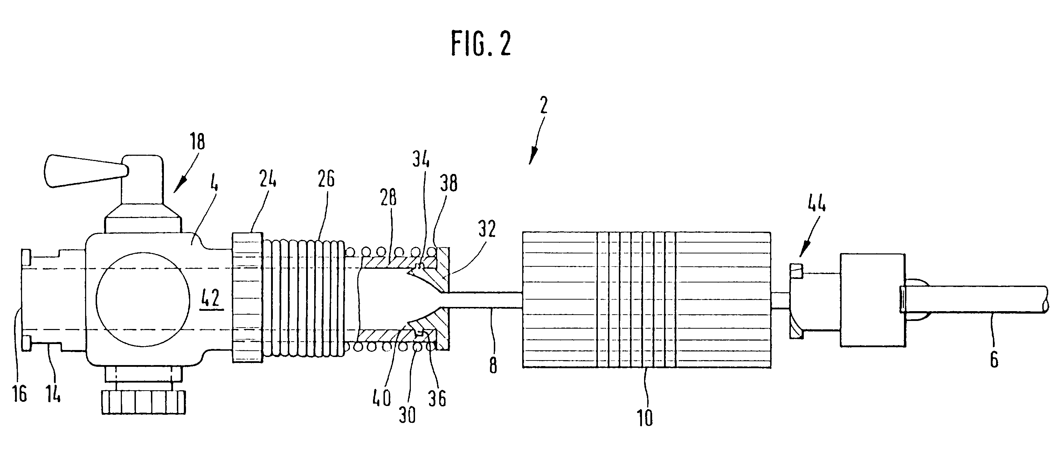

Die auseinandergegezogene Ansicht von Fig. 2 zeigt detailierter den Aufbau der Insufflationsnadel 2. So erkennt man, daß an dem Basiskörper 4 eine Befestigungshülse 24 zur Befestigung des Kanülenträgers 10 verschiebbar gelagert ist. Die Befestigungshülse 24 weist eine Befestigungseinrichtung 26 beispielsweise ein Gewinde auf, mit der eine Befestigung des Kanülenträgers 10 an der Befestigungshülse 24 erfolgt. Die Befestigungshülse 24 selbst ist gegen ein Verdrehen gegenüber dem Basiskörpers 4 beispielsweise durch einen nicht gezeigten, in einer Führungsnut verschiebbaren Stift festgelegt. Die Befestigungshülse 24 hat eine zylinderförmige Gestalt und eine durchgängige, konzentrische Bohrung und ist auf einem zylinderförmigen Führungsfortsatz 28 (dessen Außenkontur zum Teil schematisch gezeigt ist) des Basiskörpers 4 längsverschiebbar angebracht. Eine Feder 30 ist zum federnden Vorspannen der Befestigungshülse 24 und damit des Kanülenträgers 10 und der Kanüle 6 relativ zu der Insufflationslanze 8 und dem Basiskörper 4 vorgesehen.

Die Insufflationslanze 8 ist in eine Buchse 32 eingelötet. Die Buchse 32 ist mit einer Befestigungseinrichtung lösbar an dem Führungsfortsatz 28 des Basiskörpers 4 befestigt. Man erkennt die Stifte 34 und 36 eines Bajonettverschlusses, welche die Buchse 32 und damit die Insufflationalanze 8 in einer Nut in dem Führungsfortsatz 28 festlegen. An der Buchse 32 befindet sich ferner ein umlaufender Anschlag 38 für die Feder 30 sowie ein Einführkonus 40, der das Einführen der Buchse 32 in den Führungsfortsatz 28 erleichtert. Ein Austreten von Insufflationsgas zwischem dem Führungsfortsatz 28 und die Buchse 32 der Benutzung der Insufflationsnadel ist nicht erwüscht und ist durch eine Abdichtung an dieser Stelle gewährleistet.

Ein Kanal 42 durch den Basiskörper 4 ist von der Einströmöffnung 16 bis zu dem Ende des Führungsfortsatzes 28 als eine Bohrung mit konstantem Durchmesser ausgebildet. An den Kanal 42 schließt strömungsgünstig eine Passage im Inneren der Insufflationslanze an, so daß die Einströmöffnung 16 in den Basiskörper 4 mit der Ausströmöffnung 12 aus der Insufflationslanze 8 in Strömungsverbindung steht, wenn das Absperrventil 18 geöffnet ist.

Die Kanüle 6 ist mit dem Kanülenträger 10 lösbar mit einer zweigängigen Gewindeschnellverbindung, einer sogenannten LL-Verbindung, verbunden, wie bei 44 gezeigt.

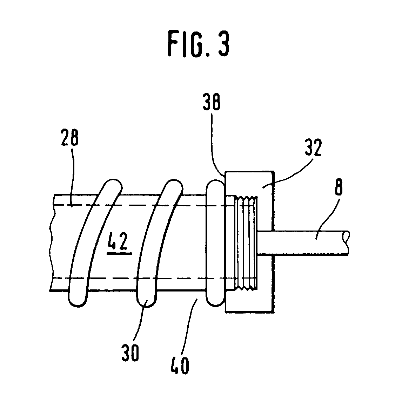

Die Fig. 3 zeigt eine alternative Möglichkeit der Befestigung der Buchse 32 und der Insufflationsnadel 8 an dem Führungsfortsatz 28 des Basiskörpers 4. Die Buchse 32 ist in der Art einer Überwurfmutter über den Führungsfortsatz 28 ausgebildet und ist durch eine Schraubverbindung an dem Führungsfortsatz 28 befestigt. Man erkennt den Anschlag 38, an dem die Feder 30 anliegt. Der Einführkonus 40 ist anders als in dem Ausführungsbeispiel der Fig. 2 als Innenkonus ausgebildet.

Die Buchse 32 kann auch dann, wenn sie wie hier als Überwurfmutter ausgebildet ist, mit einer der vorangehenden Schnellverbindungen an dem Führungsfortsatz 28 des Basiskörpers 4 befestigt sein. Ebenso kann die Buchse 32 der Fig. 2 mit einer Schraubverbindung befestigt sein. Die Buchse 32 ist ein finanziell günstig herzustellender Metalldrehkörper, in den die Insufflationslanze 8 eingelötet ist. Die Buchse 32 kann aber auch aus einem Kunststoffmaterial hergestellt sein, in das die Insufflationslanze 8 eingeklebt ist, oder das beispielsweise im Spritzgußverfahren an die Insufflationslanze 8 angegossen wurde.

## Patentansprüche

1. Insufflationsnadel (2), aufweisend:
einen Basiskörper (4) mit einer Einströmöffnung (16);
eine an dem Basiskörper (4) angebrachte, hohle Insufflationslanze (8) mit einer Ausströmöffnung (12) in ihrem von dem Basiskörper (4) entfernten Endbereich, die durch die Insufflationslanze (8) und einen Kanal (42) in dem Basiskörper (4) mit der Einströmöffnung (16) in Verbindung steht;
eine relativ zu dem Basiskörper (4) und Insufflationslanze (8) längsverschiebbar und lösbar angebrachte Kanüle (6), in der die Insufflationslanze (8) aufgenommen ist, wobei die Insufflationlanze (8) federnd so vorgespannt ist, daß im unbelasteten Zustand ihre Ausströmöffnung (12) unabgedeckt von der Kanüle (6) ist und daß im belasteten Zustand die Insufflationslanze (8) in die Kanüle (6) zurückdrückbar ist,
dadurch gekennzeichnet,
daß die Insufflationslanze (8) mit einer lösbaren Befestigungseinrichtung (34, 36) an dem Basiskörper befestigt ist.

2. Insufflationsnadel (2) nach Anspruch 1, dadurch gekennzeichnet, daß an der Insufflationslanze (8) der eine Teil der Befestigungseinrichtung (34, 36) durch Laserschweißen befestigt ist.

3. Insufflationsnadel (2) nach Anspruch 1, dadurch gekennzeichnet, daß die Insufflationslanze (8) mit einem Anschlag (38) für ein Vorspannelement (30) versehen ist, das die federnde Vorspannung liefert.

4. Insufflationsnadel (2) nach einem der Ansprüche 1 bis 3, dadurch gekennzeichnet, daß eine Schraubenfeder (30) für die federnde Vorspannung vorgesehen ist.

5. Insufflationsnadel (2) nach einem der Ansprüche 1 bis 4, dadurch gekennzeichnet, daß an der Insufflationslanze (8) eine Buchse (32) befestigt ist, an der sich der eine Teil der Befestigungseinrichtung (34, 36) befindet.

6. Insufflationsnadel (2) nach Anspruch 5, dadurch gekennzeichnet, daß der Anschlag (38) durch die Buchse (32) gebildet ist.

7. Insufflationsnadel (2) nach einem der Ansprüche 1 bis 6, dadurch gekennzeichnet, daß der Kanal (42) in dem Basiskörper (4) durchgehend bis zu der Befestigungseinrichtung (34, 36) oder der Insufflationslanze (8) einen Strömungsquerschnitt aufweist, der größer ist als der Strömungsquerschnitt der Insufflationslanze (8).

8. Insufflationsnadel (2) nach Anspruch 7, dadurch gekennzeichnet, daß der Kanal (42) eine durch den Basiskörper (4) hindurchgehende Bohrung konstanten Querschnitts ist.

9. Insufflationsnadel (2) nach einem der Ansprüche 1 bis 8, dadurch gekennzeichnet, daß die Befestigungseinrichtung eine Schraubverbindung ist.

10. Insufflationsnadel (2) nach einem der Ansprüche 1 bis 8, dadurch gekennzeichnet, daß die Befestigungseinrichtung eine Schnellverbindungseinrichtung (34, 36) ist.

11. Insufflationsnadel (2) nach einem der Ansprüche 1 bis 10, dadurch gekennzeichnet, daß der Befestigungseinrichtung ein Einführkonus (40) zugeordnet ist.

12. Insufflationanadel (2) nach einem der Ansprüche 1 bis 11, dadurch gekennzeichnet, daß die Kanüle (6) mit einer lösbaren Befestigungseinrichtung an einem Kanülenträger (10) befestigt ist, mit dem die Kanüle (6) an dem Basiskörper (4) angebracht ist.

13. Insufflationslanze (8) mit einem Teil der Befestigungseinrichtung der Insufflationsnadel (2) gemäß einem der Ansprüche 1 bis 12, wobei es sich bei der Befestigungseinrichtung um eine Schraubverbindung oder eine Schnellverbindungseinrichtung, beispielsweise eine Renkverbindung oder eine Luer Lock Schnellverbindung handelt.

14. Austauschset mit Kanüle (6) und mit Insufflationslanze (8) gemäß Anspruch 13.

15. Austauschset mit Kanüle (6), mit Insufflationslanze (8) gemäß Anspruch 13 und mit Vorspannelement (30) für die federnde Vorspannung.

## Claims

1. An insufflation needle (2), comprising:
a basic body (4) having an inflow opening (16);
a hollow insufflation lance (8) attached to the basic body (4) and having an outflow opening (12) in its end portion remote from the basic body (4), said outflow opening (12) communicating with the inflow opening (16) through the insufflation lance (8) and a channel (42) in the basic body (4);
a cannula (6) attached so as to be longitudinally displaceable and releasable in relation to the basic body (4) and the insufflation lance (8), with the insufflation lance (8) being received in said cannula (6) and being resiliently biased such that, in the unloaded state, its outflow opening (12) is uncovered by the cannula (6) and, in the loaded state, the insufflation lance (8) can be urged back into the cannula (6),
characterized in
that said insufflation lance (8) is attached to said basic body by a releasable mounting means (34, 36).

2. The insufflation needle (2) of claim 1,
characterized in that part of the mounting means (34, 36) is attached to the insufflation lance (8) by laser welding.

3. The insufflation needle (2) of claim 1,
characterized in that the insufflation lance (8) is provided with a stop (38) for a biasing member (30) providing the resilient bias.

4. The insufflation needle (2) of any of claims 1 to 3,
characterized in that a helical spring (30) is provided for said resilient bias.

5. The insufflation needle (2) of any of claims 1 to 4,
characterized in that said insufflation lance (8) has a socket (32) attached thereto at which said part of the mounting means (34, 36) is provided.

6. The insufflation needle (2) of claim 5,
characterized in that said stop (38) is formed by the socket (32).

7. The insufflation needle (2) of any of claims 1 to 6,
characterized in that the channel (42) in said basic body (4) has in continuous manner up to said mounting means (34, 36) or said insufflation lance (8), a cross-sectional area of flow which is greater than the cross-sectional area of flow of the insufflation lance (8).

8. The insufflation needle (2) of claim 7,
characterized in that the channel (42) has a bore of constant cross-section extending through said basic body (4).

9. The insufflation needle (2) of any of claims 1 to 8,
characterized in that the mounting means is a threaded connection.

10. The insufflation needle (2) of any of claims 1 to 8,
characterized in that the mounting means is a quick coupling means (34, 36).

11. The insufflation needle (2) of any of claims 1 to 10,
characterized in that the mounting means has an insertion taper (40) associated therewith.

12. The insufflation needle (2) of any of claims 1 to 11,
characterized in that the cannula (6) is attached by a releasable mounting means to a cannula carrier (10) by means of which the cannula (6) is attached to the basic body (4).

13. An insufflation lance (8) comprising a part of the mounting means for the insufflation needle (2) according to any one of claims 1 to 12, wherein said mounting means is a threaded connection or a quick coupling means, for example a bayonet-type connection or a Luer Lock quick coupling.

14. A replacement set comprising a cannula (6) and an insufflation lance (8) according to claim 13.

15. A replacement set comprising a cannula (6), an insufflation lance (8) according to claim 13 and biasing member (30) for providing the resilient bias.

## Revendications

1. Aiguille d'insufflation (2) comportant:
- un corps de base (4) avec une ouverture d'entrée (16);
- une lance d'insufflation (8) creuse, fixée au corps de base (4), avec, dans sa zone terminale éloignée du corps de base (4), une ouverture de sortie (12) qui communique avec l'ouverture d'entrée (16) par la lance d'insufflation (8) et un canal (42) ménagé dans le corps de base (4);
- une canule (6) qui peut coulisser longitudinalement par rapport au corps de base (4) et à la lance d'insufflation (8) et qui est fixée de manière amovible, dans laquelle est logée la lance d'insufflation (8), la lance d'insufflation (8) étant précontrainte élastiquement, de manière qu'à l'état non sollicité, son ouverture de sortie (12) ne soit pas recouverte par la canule (6) et de manière qu'à l'état sollicité, la lance d'insufflation (8) puisse être repoussée dans la canule (6),
caractérisée
en ce que la lance d'insufflation (8) est fixée au corps de base par un dispositif de fixation (34,36) non permanent.

2. Aiguille d'insufflation (2) selon la revendication 1, caractérisée en ce qu'une partie du dispositif de fixation (34,36) est fixée par soudage au laser à la lance d'insufflation (8).

3. Aiguille d'insufflation (2) selon la revendication 1, caractérisée en ce que la lance d'insufflation (8) est pourvue d'une butée (38) pour un élément de précontrainte (30) qui fournit la précontrainte élastique.

4. Aiguille d'insufflation (2) selon l'une des revendications 1 à 3, caractérisée en ce qu'il est prévu un ressort hélicoïdal (30) pour la précontrainte élastique.

5. Aiguille d'insufflation (2) selon l'une des revendications 1 à 4, caractérisée en ce qu'à la lance d'insufflation (8) est fixée une douille (32) sur laquelle se trouve une partie du dispositif de fixation (34,36).

6. Aiguille d'insufflation (2) selon la revendication 5, caractérisée en ce que la butée (38) est formée par la douille (32).

7. Aiguille d'insufflation (2) selon l'une des revendications 1 à 6, caractérisée en ce que le canal (42) ménagé dans le corps de base (4) présente, d'un bout à l'autre jusqu'au dispositif de fixation (34,36) ou jusqu'à la lance d'insufflation (8), une section d'écoulement qui est supérieure à la section d'écoulement de la lance d'insufflation (8).

8. Aiguille d'insufflation (2) selon la revendication 7, caractérisée en ce que le canal (42) est un perçage qui traverse le corps de base (4) d'un bout à l'autre avec une section constante.

9. Aiguille d'insufflation (2) selon l'une des revendications 1 à 8, caractérisée en ce que le dispositif de fixation est un assemblage vissé.

10. Aiguille d'insufflation (2) selon l'une des revendications 1 à 8, caractérisée en ce que le dispositif de fixation est un dispositif d'assemblage rapide (34,36).

11. Aiguille d'insufflation (2) selon l'une des revendications 1 à 10, caractérisée en ce qu'un cône d'introduction (40) est associé au dispositif de fixation.

12. Aiguille d'insufflation (2) selon l'une des revendications 1 à 11, caractérisée en ce que la canule (6) est fixée, par un dispositif de fixation non permanent, à un porte-canule (10) au moyen duquel la canule (6) est montée sur le corps de base (4).

13. Lance d'insufflation (8) avec une partie du dispositif de fixation de l'aiguille d'insufflation (2) selon l'une des revendications 1 à 12, le dispositif de fixation étant un assemblage vissé ou un dispositif d'assemblage rapide, par exemple un assemblage à baïonnette ou un raccord rapide Luer Lock.

14. Jeu de rechange avec canule (6) et avec lance d'insufflation (8) selon la revendication 13.

15. Jeu de rechange avec canule (6), avec lance d'insufflation (8) selon la revendication 13 et avec élément de précontrainte (30) pour la précontrainte élastique.
